# EUROPÄISCHE PATENTSCHRIFT

(11) **EP 1 404 261 B1**
(45) Veröffentlichungstag und Bekanntmachung des Hinweises auf die Patenterteilung: **03.01.2007**
(21) Anmeldenummer: 02715468.1
(22) Anmeldetag: 24.01.2002
(51) Int. Cl.: A61F 5/30, A61F 5/02

(54) **PELOTTE INSBESONDERE FÜR RÜCKENSTÜTZBANDAGEN**
PADS PARTICULARLY BACK-SUPPORT BANDAGES
COUSSIN D'APPUI DESTINE NOTAMMENT A DES CORSETS DE SOUTIEN DORSAL

(30) Priorität: 26.01.2001 DE 10103545
(43) Veröffentlichungstag der Anmeldung: 07.04.2004
(73) Patentinhaber: BSN Medical GmbH, 20253 Hamburg (DE)
(72) Erfinder: HERZBERG, Thorsten, 22419 Hamburg (DE)
(74) Vertreter: Harbsmeier, Jörn Felix
(86) Internationale Anmeldenummer: PCT/EP2002/000707
(87) Internationale Veröffentlichungsnummer: WO 2002/058602

(56) Entgegenhaltungen:
- EP-A- 0 619 103
- WO-A-95/32690
- DE-A- 1 816 588
- DE-U- 8 907 975
- US-A- 5 498 233

## Beschreibung

Die Erfindung betrifft Pelotten insbesondere für Rückenstützbandagen.

Orthopädische Bandagen üben entsprechend ihrer Konstruktion und ihrem Indikationsfeld eine fixierende, führende, stützende und/oder unterstützende Funktion auf die Extremitäten des menschlichen Körpers aus.
Diese medizinischen Bandagen müssen eine Form aufweisen, um den anatomischen Gegebenheiten zu entsprechen, um form- und kraftschlüssig von extern auf den menschlichen Körper einwirken zu können.

Die Herstellung von solchen medizinischen Bandagen erfolgt durch Ausschneiden von Zuschnitten aus flächigem Material, zum Beispiel aus Neopren, Gewirke, oder Geweben.
Die anatomiegerechte Form wird durch die Form der Zuschnitte oder Abnäher, zum Beispiel mit Zwickeln, und das anschließende Zusammenfügen der Zuschnitte erreicht, so wie es auch bei Bekleidung üblich ist.

Das Zusammenfügen kann durch Nähen, Kleben oder andere übliche Verfahren erfolgen. Der große Nachteil dieser Bandagen ist, daß die genaue, anatomische Paßform nur schwierig erreicht werden kann und eine Vielzahl von Verbindungsstellen entstehen, beispielsweise Nähte. Diese Verbindungsstellen verändern die Eigenschaften des eingesetzten Materials, und es besteht die Gefahr von Druckstellen auf der Haut.

Im allgemein bekannte Pelotten für Rückenbandagen weisen eine starre Form oder eine gewisse Weichheit durch eine Silikon-/Kautschuk-Beschichtung auf.

Daraus resultieren bei ersteren, bedingt durch die Starrheit der Pelotten, eine Reduzierung des Tragekomforts und eine erhöhte Neigung zu Druck und Scheuerstellen durch die harten Kanten.

Die Silikon-/Kautschuk-Pelotten zeigen einen besseren Tragekomfort auf, neigen aber durch das hohe Eigengewicht nach caudal zu rutschen. In Einzelfällen neigt die Bandage mitsamt der Pelotte nach caudal zu wandern. Ein weiteres Problem besteht darin, daß im Pelottenbereich ein vermehrtes Schwitzen beim Patienten feststellbar ist.

In der DE 36 13 235 A1 wird eine Rückenstützbandage mit einem Rückenteil und seitlichen Bauchzügen offenbart. Das Rückenteil besteht zum Beispiel aus Neopren, die Bauchzüge aus einem längselastischen Material. Die Bandage weist optional Bauchhebezüge auf, die wie die oben beschriebenen Zuggurte ausgeführt sind, und auch optional eine Rückenpelotte.

Wie der US 5,429,587, der EP 0 619 103 A1 oder der DE 27 22 563 A1 entnommen werden kann, gibt es speziell geformte Pelotten, die bei Rückenbandagen Verwendung finden.

Aus der DE 25 06 647 C1 ist eine Rückenstützbandage bekannt mit einem Zug, der durchgehend den Rückenbereich und die Seitenbereiche umfaßt und bei dem im Wirbelsäulenbereich Stützelemente vorgesehen sind.

Die US 5,498,233 zeigt eine Pelotte, die auf der dem Körper zugewandten Seite Erhebungen aufweist, wobei diese mittig eine Vertiefung aufweisen und an den jeweiligen Enden erhöhte Friktionspunkte. Dabei verlaufen die Erhebungen entlang einer geraden Linie, die senkrecht zur Längsachse der Pelotte und damit auch senkrecht zur Wirbelsäule verläuft.

Nachteilig an diesem Stand der Technik ist, dass die geradlinig verlaufenden Erhebungen als auf die Grundfläche der Pelotte aufgebrachte Verstärkungen wirken, so dass sich diese, aus dem Stand der Technik bekannte Pelotte nicht hinreichend verformen kann, um insbesondere die Zwischenwirbelmuskulatur zwischen den einzelnen Rückenwirbeln mit Druck zu beaufschlagen.

Der Erfindung liegt die Aufgabe zugrunde, eine halbstarre, funktionell modellierte Pelotte zu konzipieren, die mit einem geringen Eigengewicht einen hohen Tragekomfort aufweist, gleichzeitig aber einfach und unkompliziert für den Patienten anzulegen ist und ein hohes Maß an Dynamik aufweist. Außerdem soll die Pelotte sich hervorragend in einer Rückenbandage bewähren, die ihrerseits kostengünstig herzustellen sein soll.

Diese Aufgabe wird durch die gemäß Hauptanspruch gekennzeichnete Bandage gelöst. Gegenstand der Unteransprüche sind dabei vorteilhafte Weiterbildungen der Pelotte sowie eine mit der Pelotte ausgerüstete Rückenbandage.

Demgemäß beschreibt die Erfindung eine Pelotte insbesondere für eine Rückenbandage mit einer anterioren, dem Körper zugewandten Seite und einer posterioren, dem Körper abgewandten Seite, wobei auf der anterioren Seite der Pelotte zumindest eine damm-artige, linienförmige Erhebung vorhanden ist, die im wesentlichen parallel zur sagittalen Achse ausgerichtet ist. An den beiden Enden der dammartigen Erhebung ist zumindest ein erhöhter Friktionspunkt-vorhanden, und die zumindest eine dammartige Erhebung verläuft wellenförmig in der Ebene der Pelotte.

Der Friktionspunkt kann eine punktartige oder kreisförmige Ausgestaltung aufweisen. In Verbindung mit der bevorzugten Ausgestaltung der Erhebungen nach lateral mit unterschiedlichen Höhen der Profilierung führt dies zu der bevorzugt gewollten Friktion der medialen und lateralen Stränge der Rückenmuskulatur.

In einer weiteren vorteilhaften Weiterbildung der Pelotte verläuft die dammartige Erhebung spiegelsymmetrisch zur sagittalen Achse.

In einer weiteren vorteilhaften Weiterbildung der Pelotte sind auf dieser untereinander drei dammartige Erhebungen angeordnet.

Zur Herstellung erfindungsgemäßer Pelotten können unterschiedliche Materialien Verwendung finden, so zum Beispiel Abstandsgewebe, Schaumstoffe, Vliese und vergleichbare Materialien.

Abstandsgewebe werden in der EP 0 071 212 B1 offenbart. Abstandsgewebe sind mattenförmige Schichtkörper mit einer Deckschicht aus einem Faser- oder Filamentvlies, einer Unterlagsschicht und zwischen diesen Schichten vorhandene einzelne oder Büschel von Haltefasern, die über die Fläche des Schichtkörpers verteilt durch die Partikelschicht hindurchgenadelt sind und die Deckschicht und die Unterlagsschicht untereinander verbinden. Als zusätzliches, aber nicht erforderliches Merkmal sind gemäß EP 0 071 212 B1 in den Haltefasern Partikel aus inerten Gesteinspartikeln, wie zum Beispiel Sand, Kies oder dergleichen, vorhanden.

Die durch die Partikelschicht hindurchgenadelten Haltefasern halten die Deckschicht und die Unterlagsschicht in einem Abstand voneinander, und sie sind mit der Deckschicht und der Unterlagsschicht verbunden.
Abstandsgewebe oder -gewirke sind u. a. in zwei Artikeln beschrieben, und zwar einem Artikel aus der Fachzeitschrift "kettenwirk-praxis 3/93", 1993, Seiten 59 bis 63 "Raschelgewirkte Abstandsgewirke"
und
einem Artikel aus der Fachzeitschrift "kettenwirk-praxis 1/94", 1994, Seiten 73 bis 76 "Raschelgewirkte Abstandsgewirke".

Des weiteren können erfindungsgemäß Verwendung zur Herstellung von Pelotten finden Materialien mit unterschiedlichen Festigkeiten, Raumgewichten usw.

In einer weiteren vorteilhaften Weiterbildung der Pelotte ist die Pelotte ein-, beidseitig oder vollständig von einem Textilgewebe umschlossen, um den Tragekomfort zu erhöhen.

In einer weiteren vorteilhaften Weiterbildung der Pelotte ist die Pelotte perforiert, zum Beispiel mit mehreren, zu einem Muster angeordneten Löchern mit einem Durchmesser von 1 bis 5 mm, um die Atmungsaktivität der Pelotte zu steigern.

Erfindungsgemäße Pelotten können nach unterschiedlichen Verfahren hergestellt werden.

Sind die Pelotten aus Schaumgummi, können diese durch Kompressionsmolding zu unterschiedlichen Formen und Dicken gepreßt werden. Dadurch sollen durch die unterschiedliche Dichte des Schaumstoffs nach der Verformung die elastischen Eigenschaften des Materials lokal verändert werden. Hierzu wird auf die WO 95/32690 A1 verwiesen, in der das Verfahren ausführlich beschrieben ist.

Weiterhin ist es möglich, thermoplastisches Kunststoffplattenmaterial anatomisch formgerecht zu den erfindungsgemäßen Pelotten zu formen. Diese Materialien, wie beispielsweise Polyethylen (HDPE), Polypropylen oder ein Copolymer PP, besitzen einen thermoplastischen Umformungsbereich von ca. 170 °C bis 250 °C.

Besonders vorteilhaft lassen sich Pelotten herstellen durch dreidimensionales thermisches Anpassen, deren Ausgangsmaterial zu mindestens 10 Gew.-% aus thermoplastischem Material besteht.

Das Ausgangsmaterial ist dabei vorzugsweise ein thermoplastisch verformbares Abstandsgewirk, ein thermoplastisch verformbarer Vliesstoff, ein thermoplastisch verformbarer Schaumstoff und/oder ein thermoplastisch verformbarer Kunststoff mit einer geringen Rigidität.
Mögliche Kunststoffe, die eine solche geringe Rigidität aufweisen, sind Polyethylen (LDPE) und Polypropylen.

Weiter vorzugsweise ist das Ausgangsmaterial ein thermoplastisch verformbares Gewebe, Gewirke oder Gestrick, wobei jeweils elastische Fasern oder Komponenten integriert sein können.
Die aufgeführten Materialien können dabei durch die Einarbeitung von beispielsweise Elastan oder Elstodien derartig elastisch sein.

Weiter vorzugsweise ist das Ausgangsmaterial zu zwei- oder mehrlagigen Laminaten verbunden, wobei das Laminieren mittels allgemein bekannter Verfahren erfolgt, zum Beispiel dem Kaschieren.

Eine erfindungsgemäße Pelotte kann vorteilhafterweise dadurch hergestellt werden, daß das auf entsprechende Paßform zugeschnittene Ausgangsmaterial in den thermoplastischen Eniveichungsbereich erhitzt wird und anschließend das Ausgangsmaterial mittels einer entsprechend geformten Positivform in die benötigte Form gepreßt wird.

Weiter vorteilhafterweise erfolgt die Herstellung der Pelotte dadurch, daß das auf entsprechende Paßform zugeschnittene Ausgangsmaterial zwischen einer Positivform und einer Negativform, die entsprechend geformt sind, in den thermoplastischen Erweichungsbereich erhitzt und in die benötigte Form gepreßt wird.
Das Erhitzen kann mittels beheizbarer Formen erfolgen oder auch, indem das Ausgangsmaterial zunächst in einem Ofen entsprechend erwärmt wird und dann erst in den Formen geformt wird.

Als besonders -vorteilhaft hat -sich die Verwendung eines weicheren -Schaumstoffes zur Körperseite und eine härtere Variante zur Bandage erwiesen, um so den Tragekomfort hautseitig zu erhöhen.

Durch die halbstarre Konstruktion wird eine optimale Anpassung der Pelotte insbesondere am Rücken des Trägers erreicht, bei gleichzeitiger stützender Wirkung auf die überdeckenden Wirbelsäulenpartie.

Sodann umfaßt der erfinderische Gedanke eine Rückenstützbandage mit zumindest einer der erfindungsgemäßen Pelotten.

In einer ersten vorteilhaften Ausführungsform der Rückenstützbandage weist diese ein Rückenteil, seitliche Bauchzüge und einen Verschluß, insbesondere einen Klettverschluß, zum Verbinden der Bauchzüge auf, wobei das Rückenteil aus einem im wesentlichen unelastischen Material und die Bauchzüge aus einem im wesentlichen einzügigen, insbesondere elastischen Material gebildet sind.

Vorzugsweise erstreckt sich die Dehnungsrichtung des die Bauchzüge bildenden Materials von dem Rückenteil zu den Verschlußteilen.

Weiter vorzugsweise weist das Rückenteil auf seiner der Wirbelsäule zuzuwendenden Innenseite ein Klettband zum Aufnehmen der auswechselbaren Pelotten auf.

Des weiteren können die Bauchzüge an ihren die Verschlußteile aufnehmenden Seiten Abdominalverstärkungen aufweisen, die wiederum insbesondere als dünne Kunststoffplatten beziehungsweise -stege ausgebildet sind.

In einer weiteren bevorzugten Ausführungsform der Rückenbandage sind auf der der Wirbelsäule abgewandten Außenseite sich über die Bauchzüge hinwegerstreckende. Bauchhebezüge vorgesehen, die wiederum vorteilhaft aus einem im wesentlichen unelastischen Material gebildet sind.

In einer weiteren bevorzugten Ausführungsform der Rückenbandage sind im Rückenteil sich im wesentlichen parallel zur Wirbelsäule-erstreckende Verstärkühgsstäbe vorgesehen.

Diese Rückenbandage ermöglicht eine hohe Formschlüssigkeit zwischen der zu haltenden Pelotte und den Wirbelsäulensegmenten einerseits und einen guten Sitz und eine flächige Anstützung über dem Bauchbereich, des weiteren eine gute Stabilisierung der Lendenwirbelsegmente und des Kreuzbeines.

Die Erfindung wird anhand von zwei Figuren eines Ausführungsbeispiels näher erläutert, ohne damit die Erfindung unnötig einschränken zu wollen.

Es zeigen
- Figur 1: die Pelotte in anteriorer Ansicht und
- Figur 2: die Pelotte in posteriorer Ansicht.

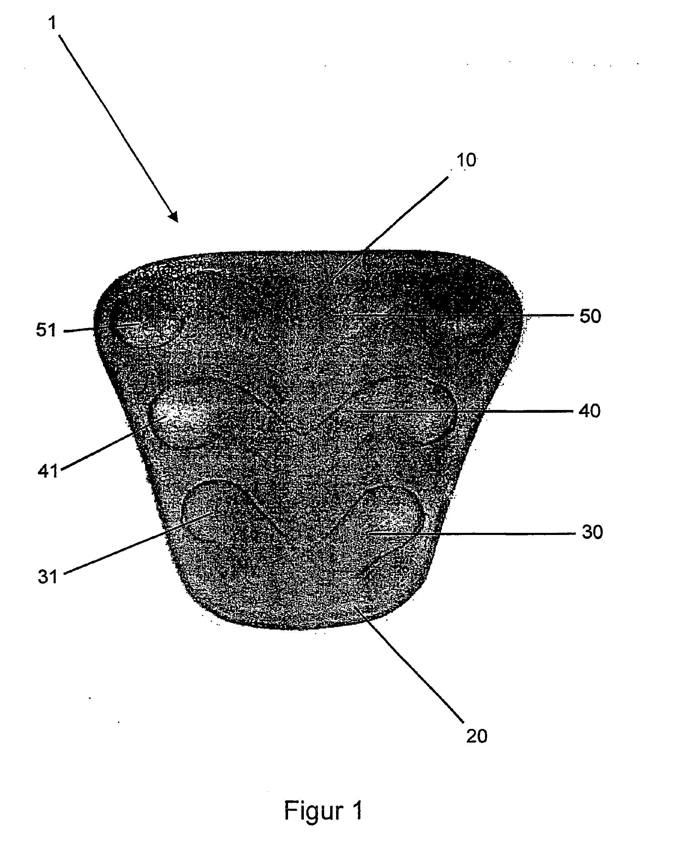

In der Figur 1 ist die Pelotte 1 insbesondere für eine Rückenbandage in einer besonders vorteilhaften Ausführungsform gezeigt, und zwar in anteriorer Ansicht.
Die Pelotte 1 besteht aus einer anterioren, dem Körper zugewandten Seite und einer posterioren, dem Körper abgewandten Seite, wobei auf der anterioren Seite der Pelotte 1 drei dammartige, linienförmige Erhebungen 30, 40, 50 vorhanden sind, die im wesentlichen parallel zur horizontalen Achse ausgerichtet sind. An jeweils den beiden Enden der dammartigen Erhebungen 30, 40, 50 ist ein erhöhter Friktionspunkt 31, 41, 51 vorhanden.

Die linienförmigen Erhebungen 30, 40, 50 sind dabei in Form einer Welle ausgeformt. Des weiteren weisen diese nach lateral eine zunehmende Höhe in der Profilierung auf.

Die Pelotte 1 ist im wesentlichen trapezförmig geformt, wobei sich die Pelotte vom oberen Bereich 10 zum unteren Bereich 20 verjüngt.

Die linienförmigen Erhebungen 30, 40, 50 weisen mittig eine Höhe von 1,5 bis 12 mm; bevorzugt 4,5 bis 8 mm, sowie eine Breite von 10 bis 30 mm, bevorzugt 12 bis 25 mm, auf.
Der Friktionspunkt 31, 41, 51 weist einen Durchmesser von 10 bis 40 mm, bevorzugt 20 bis 30 mm, sowie eine Höhe von 2 bis 20 mm, bevorzugt 4,5 bis 10 mm, auf.

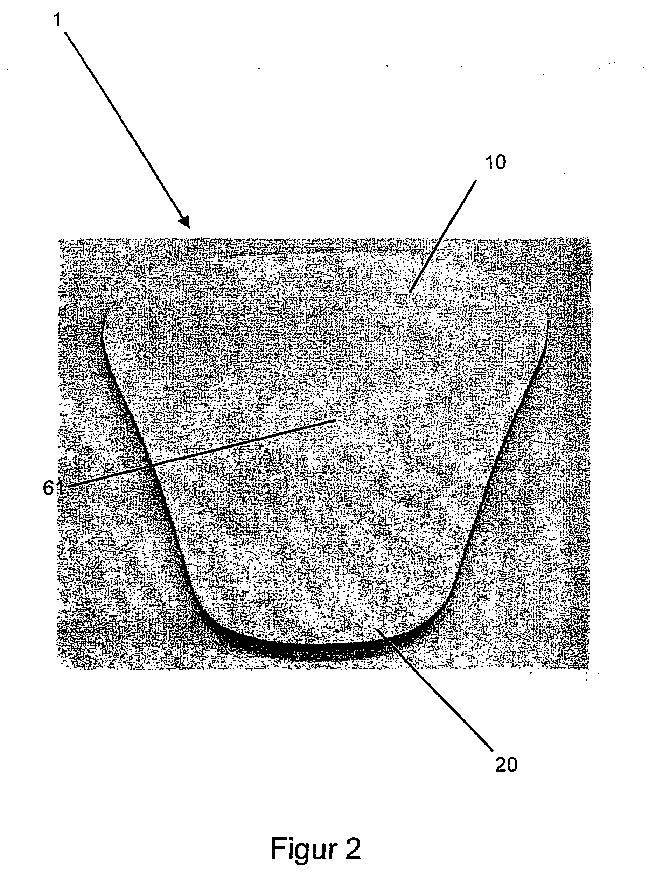

Figur 2 zeigt die Pelotte 1 in posteriorer Ansicht. Mittig auf der Pelotte 1 ist ein Klettstreifen 61 angebracht, der dazu dient, die Pelotte 1 an einer Rückenbandage zu befestigen. Die Pelotte 1 ist an der dem Körper zugewandten Seite mit einer weichen, flüssigkeitsaufnehmenden Schicht eines Gewebes eingedeckt, um den Komfort des Patienten zu erhöhen.

## Patentansprüche

1. Pelotte insbesondere für eine Rückenbandage mit einer anterioren, dem Körper zugewandten Seite und einer posterioren, dem Körper abgewandten Seite, wobei auf der anterioren Seite der Pelotte zumindest eine dammartige, linienförmige Erhebung (30, 40, 50) vorhanden ist, die im wesentlichen parallel zu sagittalen Achse ausgerichtet ist, wobei an den beiden Enden der dammartigen Erhebung (30, 40, 50) zumindest ein erhöhter Friktionspunkt (31, 41, 51) vorhanden ist, **dadurch gekennzeichnet, daß** die zumindest eine dammartige Erhebung (30, 40, 50) wellenförmig in der Ebene der Pelotte verläuft.

2. Pelotte nach Anspruch 1, **dadurch gekennzeichnet, daß** die dammartige Erhebung (30, 40, 50) spiegelsymmetrisch zur sagittalen Achse verläuft.

3. Pelotte nach den Ansprüchen 1 und 2, **dadurch gekennzeichnet, daß** auf der Pelotte untereinander drei dammartige Erhebungen (30, 40, 50) angeordnet sind.

4. Pelotte nach zumindest einem der vorherigen Ansprüche, **dadurch gekennzeichnet, daß** das Ausgangsmaterial für die Pelotte ein thermoplastisch verformbares Abstandsgewirk, ein thermoplastisch verformbarer Vliesstoff, ein thermoplastisch verformbarer Schaumstoff und/oder ein thermoplastisch verformbarer Kunststoff mit einer geringen Rigidität ist.

5. Pelotte nach zumindest einem der vorherigen Ansprüche, **dadurch gekennzeichnet, daß** das Ausgangsmaterial für die Pelotte ein thermoplastisch verformbares Gewebe, Gewirke oder Gestrick ist, in das elastische Fasern oder Komponenten integriert sein können.

6. Verfahren zur Herstellung einer Pelotte nach zumindest einem der vorherigen Ansprüche, **dadurch gekennzeichnet, daß** das Ausgangsmaterial durch dreidimensionales thermisches Anpassen an die Anatomie des vorgegebenen Körperteils angepaßt wird.

7. Verfahren zur Herstellung einer Pelotte nach zumindest einem der vorherigen Ansprüche, **dadurch gekennzeichnet, daß** das auf entsprechende Paßform zugeschnittene Ausgangsmaterial in den thermoplastischen Erweichungsbereich erhitzt wird und das Ausgangsmaterial mittels einer entsprechend den anatomischen Gegebenheiten des jeweiligen Körperteils geformte Positivform in die benötigte Form gepreßt wird.

8. Verfahren zur Herstellung einer Pelotte nach zumindest einem der vorherigen Ansprüche, **dadurch gekennzeichnet, daß** das auf entsprechende Paßform zugeschnittene Ausgangsmaterial zwischen einer Positivform und einer Negativform, die entsprechend den anatomischen Gegebenheiten des jeweiligen Körperteils geformt sind, in den thermoplastischen Erweichungsbereich erhitzt und in die benötigte Form gepreßt wird.

9. Rückenstützbandage mit zumindest einer der Pelotten nach zumindest einem der vorherigen Ansprüche.

10. Rückenstützbandage nach Anspruch 9 mit einem Rückenteil und seitlichen, insbesondere elastischen Bauchzügen und einem Verschluß zum Verbinden der Bauchzüge, wobei das Rückenteil aus einem im wesentlichen unelastischen Material und die Bauchzüge aus einem im wesentlichen einzügigen Material gebildet sind.

## Claims

1. Pad, in particular for a back bandage, having an anterior side facing the body and a posterior side facing away from the body, there being on the anterior side of the pad at least one dam-shaped linear elevation (30, 40, 50), which is oriented substantially parallel to the sagittal axis, and there being at least one raised friction point (31, 41, 51) at the two ends of the dam-shaped elevation (30, 40, 50), **characterized in that** the at least one dam-shaped elevation (30, 40, 50) extends in the shape of a wave in the plane of the pad.

2. Pad according to Claim 1, **characterized in that** the dam-shaped elevation (30, 40, 50) extends mirror-symmetrically in relation to the sagittal axis.

3. Pad according to Claims 1 and 2, **characterized in that** three dam-shaped elevations (30, 40, 50) are arranged on the pad, one below another.

4. Pad according to at least one of the preceding claims, **characterized in that** the raw material for the pad is a thermoplastically deformable spacer knit, a thermoplastically deformable nonwoven, a thermoplastically deformable foam material, and/or a thermoplastically deformable plastic of low rigidity.

5. Pad according to at least one of the preceding claims, **characterized in that** the raw material for the pad is a thermoplastically deformable fabric or knit, into which elastic fibres or components may be integrated.

6. Method of producing a pad according to at least one of the preceding claims, **characterized in that** the raw material is adapted to the anatomy of the predetermined body part by means of a three-dimensional heat fitting operation.

7. Method of producing a pad according to at least one of the preceding claims, **characterized in that** the raw material tailored to the appropriate fit is heated to the thermoplastic softening range, and the raw material is pressed to the required shape by means of a positive mould that is formed in accordance with the anatomical conditions of the particular body part.

8. Method of producing a pad according to at least one of the preceding claims, **characterized in that** the raw material tailored to the appropriate fit is heated to the thermoplastic softening range between a positive mould and a negative mould, which are formed in accordance with the anatomical conditions of the particular body part, and pressed to the required shape.

9. Back support bandage having at least one of the pads according to at least one of the preceding claims.

10. Back support bandage according to Claim 9, having a back portion and lateral, in particular elastic, abdominal bands and a closure for joining the abdominal bands, the back portion being formed of a substantially inelastic material and the abdominal bands of a substantially single-stretch material.

## Revendications

1. Pelote, en particulier pour un bandage dorsal, avec une face antérieure tournée vers le corps et une face postérieure détournée du corps, et où, sur la face antérieure de la pelote, il existe au moins une saillie linéaire en forme de digue (30, 40, 50) qui est orientée essentiellement parallèlement à l'axe sagittal, et où, aux deux extrémités de la saillie en forme de digue (30, 40, 50), il y a au moins un point de friction: surélevé (31, 41, 51), **caractérisée en ce que** l'au moins une saillie en forme de digue (30, 40, 50) s'étend en forme de vagues dans le plan de la pelote.

2. Pelote selon la revendication 1, **caractérisée en ce que** La saillie en forme de digue (30, 40, 50) s'étend selon une symétrie spéculaire par rapport à l'axe sagittal.

3. Pelote selon les revendications 1 et 2, **caractérisée en ce que**, sur la pelote, trois saillies en forme de digue (30, 40, 50) sont disposées l'une au-dessous l'autre.

4. Pelote selon l'une au moins des revendications précédentes, **caractérisée en ce que** le matériau de départ pour la pelote est un tricot intercalaire thermoplastiquement déformable, un non-tissé thermoplastiquement déformable, une mousse thermoplastiquement déformable et/ou un plastique thermoplastiquement déformable avec une faible rigidité.

5. Pelote selon l'une au moins des revendications précédentes, **caractérisée en ce que** le matériau de départ pour la pelote est un tissu ou un tricot ou un tricotage thermoplastiquement déformable, dans lequel peuvent être incorporés des fibres ou des composants élastiques.

6. Procédé de fabrication d'une pelote selon l'une au moins des revendications précédentes, **caractérisé en ce que** le matériau de départ est adapté par ajustement thermique tridimensionnel à l'anatomie de la partie de corps donnée.

7. Procédé de fabrication d'une pelote selon l'une au moins des revendications précédentes, **caractérisé en ce que** le matériau de départ, taillé à la forme adaptée correspondante, est chauffé dans le domaine de ramollissement thermoplastique, et **en ce que** le matériau de départ est pressé dans la forme nécessaire au moyen d'un moule positif façonné en fonction des données anatomiques de la partie du corps respective.

8. Procédé de fabrication d'une pelote selon l'une au moins des revendications précédentes, **caractérisé en ce que** le matériau de départ, taillé à la forme adaptée correspondante, est chauffé dans le domaine de ramollissement thermoplastique entre un moule positif et un moule négatif, qui sont façonnés en fonction des données anatomiques de la partie du corps respective, et est pressé dans la forme nécessaire.

9. Bandage de soutien dorsal avec au moins une des pelotes selon l'une au moins des revendications précédentes.

10. Bandage de soutien dorsal selon la revendication 9 avec une partie dorsale et des sangles abdominales latérales, en particulier élastiques, et une boucle pour relier les sangles abdominales, sachant que la partie dorsale est formée d'un matériau essentiellement inélastique et les sangles abdominales en un matériau essentiellement rétractable.
